# EUROPÄISCHE PATENTSCHRIFT

(11) **EP 3 596 034 B1**
(45) Veröffentlichungstag und Bekanntmachung des Hinweises auf die Patenterteilung: **02.12.2020**
(21) Anmeldenummer: 18715470.3
(22) Anmeldetag: 15.03.2018
(51) Int. Cl.: C07C 29/00, C07C 29/60, C07C 31/20

(54) **VERFAHREN ZUR CHEMISCHEN UMSETZUNG VON ZUCKERN ODER ZUCKERALKOHOLEN ZU GLYKOLEN**
METHOD FOR CHEMICAL CONVERSION OF SUGARS OR SUGAR ALCOHOLS TO GLYCOLS
PROCÉDÉ DE TRANSFORMATION CHIMIQUE DE SUCRES OU D'ALCOOLS DE SUCRE EN GLYCOLS

(30) Priorität: 15.03.2017 DE 102017204322
(43) Veröffentlichungstag der Anmeldung: 22.01.2020
(73) Patentinhaber: thyssenkrupp AG, 45143 Essen (DE); thyssenkrupp Industrial Solutions AG, 45143 Essen (DE)
(72) Erfinder: GLOTZBACH, Christoph, 32052 Herford (DE); SCHIRRMEISTER, Steffen, 45472 Mülheim an der Ruhr (DE); PALKOVITS, Regina, 52074 Aachen (DE); HAUSOUL, Peter, 6371 ETLandgraaf (NL); BEINE, Anna Katharina, 52074 Aachen (DE)
(74) Vertreter: thyssenkrupp Intellectual Property GmbH
(86) Internationale Anmeldenummer: PCT/DE2018/100236
(87) Internationale Veröffentlichungsnummer: WO 2018/166566

(56) Entgegenhaltungen:
- WO-A1-2008/071642
- WO-A1-2016/119568
- DE-A1-102008 028 070

## Beschreibung

Die vorliegende Erfindung betrifft ein Verfahren zur chemischen Umsetzung von Zuckern oder Zuckeralkoholen zu Polyolen/Glykolen.

Die Herstellung von Basis- und Feinchemikalien sowie die Gewinnung von Energie aus Erdöl, Kohle und Erdgas ist Stand der Technik. Diese Kohlenstoffquellen sind jedoch oftmals schwer zugänglich und nicht erneuerbar. Die Gewinnung und Verarbeitung fossiler Rohstoffe ist energieintensiv und produziert Klimagase in erheblichen Mengen. Eine nachhaltige und CO₂-neutrale Alternative bildet die Verwendung von Biomasse. Der Aufschluss pflanzlicher Biomasse erfolgt mittels Fermentation oder anderer Verfahren. Zur Wertschöpfung aus den Bestandteilen der Biomasse ist eine weitere Aufarbeitung der Spaltprodukte nötig. Die chemische Umwandlung biobasierter Zucker und Zuckeralkohole zu Glykolen ist deshalb Gegenstand der vorliegenden Erfindung.

Im klassischen Verfahren erfolgt die Herstellung von Ethylenglykol aus Ethylen, welches zu Ethylenoxid umgesetzt und dann zu Ethylenglykol hydratisiert wird. Propylenglykol wird herkömmlicherweise durch Hydratisierung von Propylenoxid hergestellt.

Die Umsetzung von Zuckern und Zuckeralkoholen zu Glykolen ist ebenfalls aus der Literatur bekannt. Beispielsweise wird in der WO 03/035593 A1 ein Verfahren zur Umsetzung von 5-C Zuckern und Zuckeralkoholen mit Wasserstoff bei erhöhten Temperaturen von mehr als 120 °C, basischem pH-Wert und in Gegenwart eines Rhenium haltigen Katalysators, der außerdem Nickel enthält beschrieben. Dabei tritt eine Hydrogenolyse sowohl von C-C-Bindungen, als auch von CO-Bindungen auf, so dass die 5-C Zucker und Zuckeralkohole gespalten werden und über Zwischenstufen als Produkt Propylenglykol (CH₃-CHOH-CH₂OH) entsteht. Als alternative metallische Katalysatoren werden auch Ru, Pt, Pd, Ir und Rh genannt.

Als Nebenprodukte in der Hydrogenolyse zu Ethylenglykol und Propylenglykol treten Glycerin und Milchsäure auf. Die Herausforderung bei dem vorgenannten Verfahren liegt daher in der Unterdrückung der Milchsäurebildung und der Optimierung der Glykolselektivität.

Stickstoffhaltige Kohlenstoffträger und Carbon Nanotubes (CNTs) können über verschiedene Synthesewege erhalten werden und weisen je nach Herstellung unterschiedliche Stickstoffgehalte auf. Sie werden hauptsächlich für die Katalyse von Oxidationsreaktionen, Gasadsorption und in der Elektrochemie verwendet.

In der US 2010/0276644 A1 wird ein Verfahren zur Herstellung von mit Stickstoff dotierten Carbon Nanotubes beschrieben, bei dem zunächst ein Metall aus einer Lösung eines Metallsalzes in einem Solvens ausgefällt wird, so dass eine Suspension erhalten wird, aus der der Feststoff abgetrennt wird, wobei ein heterogener Metallkatalysator erhalten wird. Dieser Katalysator wird in ein Fließbett eingebracht, in dem man ihn mit einem Kohlenstoff und Stickstoff haltigen Material reagieren lässt, wodurch man die mit Stickstoff dotierten Kohlenstoff-Nanotubes erhält. Das Metallsalz, von dem ausgegangen wird, ist bevorzugt ein Salz des Kobalts, Mangans, Eisens oder Molybdäns. Der heterogene Metallkatalysator enthält außerdem Al₂O₃ und MgO. Das Kohlenstoff und Stickstoff haltige Material ist beispielsweise eine organische Verbindung, die in gasförmigem Zustand vorliegt und welche zum Beispiel ausgewählt werden kann aus Acetonitril, Dimethylformamid, Ácrylnitril, Propionitril, Butyronitril, Pyridin, Pyrrol, Pyrazol, Pyrrolidin und Piperidin. In der genannten US-Schrift 2010/0276644 A1 wird die Verwendung der Carbon Nanotubes als Additive zur mechanischen Verstärkung von Materialien vorgeschlagen sowie zur Erhöhung deren elektrischer Leitfähigkeit oder thermischer Leitfähigkeit. Beispielsweise eignen sich die Stickstoff dotierten Carbon Nanotubes für die Herstellung von Leiterbahnen, Batterien, oder Beleuchtungseinrichtungen oder als Speichermedium für Wasserstoff oder Lithium in Membranen. Weiter werden Anwendungen in Brennstoffzellen oder im medizinischen Bereich als Unterlagen für die Kontrolle des Wachstums von Zellgewebe genannt. In dieser Druckschrift wird somit ein sehr breites Spektrum von Anwendungen in den unterschiedlichsten Bereichen genannt.

In der WO 2016/119568 A1 wird ein Heteroatome enthaltendes Nanokohlenstoff-Material sowie dessen Herstellung beschrieben. Das Material enthält bis zu 2 Gew.-% Stickstoff und 1 bis 6 Gew.-% Sauerstoff. Das dort beschriebene Nanokohlenstoff-Material soll gute katalytische Eigenschaften bei der Dehydrierung von Kohlenwasserstoffen aufweisen.

WO 2008/071642 A1 beschreibt die Hydrogenolyse von Sorbit unter Verwendung eines Palladium- oder Rutheniumkatalysators.

Die Aufgabe der vorliegenden Erfindung besteht darin, ein Verfahren zur chemischen Umsetzung von Zuckern oder Zuckeralkoholen zu Glykolen mit den Merkmalen der eingangs genannten Gattung zur Verfügung zu stellen, welches die Herstellung von Glykolen mit höherer Selektivität ermöglicht und die Bildung von Milchsäure als Nebenprodukt verringert.

Die Lösung der vorgenannten Aufgabe liefert ein Verfahren der eingangs genannten Art mit den Merkmalen des Anspruchs 1.

Erfindungsgemäß ist vorgesehen, dass als Katalysator-Träger ein mit Stickstoff dotierter Kohlenstoff-Träger verwendet wird.

Eine bevorzugte Weiterbildung der erfindungsgemäßen Aufgabenlösung sieht vor, dass als Katalysator-Träger ein mit Stickstoff dotierter Kohlenstoffträger, insbesondere mit Stickstoff dotierte Aktivkohle, verwendet wird. Alternativ kann beispielsweise mit Stickstoff dotierter Ruß als Kohlenstoffträger verwendet werden.

Unter einem mit Stickstoff dotierten Kohlenstoffträger wird im Rahmen dervorliegenden Erfindung verstanden: Ein Kohlenstoffträger, dessen Oberfläche eine Stickstoffdotierung aufweist. Die Herstellung dieser mit Stickstoff dotierten Kohlenstoffträger kann mittels geeigneter Vorgängermaterialien bei der Herstellung des Kohlenstoffträgers an sich, als auch nachträglich zum Beispiel mittels reduktiver Methoden erfolgen. Zwei mögliche Verfahren, die im Rahmen der vorliegenden Erfindung angewendet wurden, werden in den Beispielen beschrieben.

Gemäß einer alternativen bevorzugten Variante des erfindungsgemäßen Verfahrens werden als Katalysator-Träger mit Stickstoff dotierte Carbon Nanotubes verwendet.

Mit Stickstoff dotierte Carbon Nanotubes sind gemäß dervorliegenden Erfindung wie folgt definiert:
Zylinderförmige Kohlenstoffhohlkörper mit einem Durchmesser von 3 bis 90 nm, die vor, während oder nach der Herstellung des Kohlenstoffhohlkörpers mit Stickstoff dotiert wurden.

Vorzugsweise wird als Co-Katalysator eine Base verwendet. Dabei kommen im Rahmen der vorliegenden Erfindung insbesondere die folgenden Basen in Betracht:
Alle Alkalihydroxide, insbesondere Natriumhydroxid (NaOH), Kaliumhydroxid (KOH) und Lithiumhydroxid (LiOH).
Alle Erdalkalihydroxide, insbesondere Magnesiumhydroxid (Mg(OH)₂), Calciumhydroxid (Ca(OH)₂), Strontiumhydroxid (Sr(OH)₂) und Bariumhydroxid (Ba(OH)₂).

Wenn man in dem erfindungsgemäßen Verfahren von einem Zucker ausgeht, liegt ein zweistufiger Prozess vor, wobei zunächst der Zucker in an sich bekannter Weise zu den Zuckeralkoholen hydriert wird und anschließend in einem zweiten Schritt mit einem Katalysator die Hydrogenolyse der Zuckeralkohole, die bei der Hydrierung der Zucker entstehen, zu den Polyolen stattfindet. Dabei ist es generell auch möglich, beide genannten Umsetzungsprozesse in nur einem Schritt zu vollziehen, oder in einer Reaktionssequenz in einem Reaktor, so dass die Zucker direkt zu den Polyolen umgesetzt werden. Bei dieser Variante sind die Ausbeuten schlechter, da verschiedene sich störende Reaktionsmechanismen nebeneinander ablaufen.

Das erfindungsgemäße Verfahren kommt insbesondere für die Hydrierung mit anschließender Hydrogenolyse folgender Zucker und der dabei entstehenden Zuckeralkohole in Betracht:
C5-Zucker, beispielsweise die nachfolgend genannten Verbindungen:
   Ribose, Arabinose, Xylose, Lyxose;
C5-Zuckeralkohole, beispielsweise die nachfolgend genannten Verbindungen:
   Ribitol, Arabitol, Xylitol, Lyxitol;
C6- und andere Zucker sowie -Zuckeralkohole, beispielsweise:
   Allose, Altrose, Glucose, Mannose, Gulose, Idose, Galactose, Talose, Allitol, Talitol, Sorbitol, Mannitol, Iditol, Fucitol, Galactitol, Erythritol, Threitol, Glycerin.

Bei der Hydrogenolyse von C5-Zuckern entstehen die nachfolgend genannten Produkte:
Xylitol, Ribitol, Arabitol, Lyxitol.

Bei der Hydrogenolyse von C5-Zuckeralkoholen entstehen die nachfolgend genannten Spaltprodukte: Glycerin, Ethylenglycol, Propylenglycol, Milchsäure, Glykolsäure sowie bei bestimmten Reaktionsbedingungen auch Erythritol, und Anhydroxylitol.

Vorzugsweise erfolgt gemäß einer Weiterbildung der Erfindung die Umsetzung bei einer Reaktionstemperatur im Bereich von etwa 170 °C bis 200 °C.

Das vorgenannte Temperaturintervall ist vorteilhaft, denn wenn man niedrigere Temperaturen wählt, dann findet eine Reaktion sehr langsam bzw. gar nicht statt.

Wählt man höhere Temperaturen, dann kommt es unter anderem vermehrt zu Deoxygenierungs- und Decarbonylierungsreaktionen, sowie zu Cyclisierungen. Es entstehen deutlich mehr Nebenprodukte, wie z.B. Erythritol, Threitol und Anhydroxylitol.

Gemäß einer bevorzugten Weiterbildung des erfindungsgemäßen Verfahrens erfolgt die Hydrogenolyse bei einem Wasserstoffdruck im Bereich von etwa 50 bar bis 80 bar.

Die vorgenannten Wasserstoffdrücke haben sich als besonders vorteilhaft erwiesen, denn wenn man niedrigere Drücke wählt, dann entstehen mehr Nebenprodukte. Eine Carbonylbildung wird bevorzugt.

Wählt man Wasserstoffdrücke oberhalb des genannten Bereichs, dann hat dies den Nachteil, dass die Reaktion zum einen industriell schwer umzusetzen ist und zum anderen wird die Umsetzung des Edukts deutlich verlangsamt. Beispielsweise kann im Rahmen der vorliegenden Erfindung der Katalysator als Metall Ruthenium und/oder Platin und/oder Nickel enthalten. Weiterhin kommen die restlichen Elemente der Platingruppe (Os, Rh, Ir, Pd), sowie Au, Ni, Cu, Fe und Co in Betracht. Dabei kann der erfindungsgemäße Katalysator eines oder mehrere der genannten Metalle enthalten.

Nachfolgend wird die vorliegende Erfindung anhand von Ausführungsbeispielen unter Bezugnahme auf die beiliegenden Zeichnungen näher beschrieben. Dabei zeigen:
Figur 1 die Produktentwicklung über die Zeit für N-600,5-Ru (Bedingungen: T = 200 °C, p(H₂) = 80 bar, m(Kat) = 0.1563 g, m(Xylitol) = 1.50 g, m(Ca(OH)₂) = 0.225 g, 15 mL H₂O);
Figur 2 die Produktentwicklung über die Zeit für C-Ru;
Figur 3 einen Vergleich der Katalysatoren (Bedingungen: T = 200 °C, p(H₂) = 80 bar, m(Ru) = 0.01 g, m(Xylitol) = 1.50 g, m(Ca(OH)₂) = 0.225 g, 15 mL H₂O);
Figur 4 ein Metallscreening auf stickstoffhaltigen Kohlen (Bedingungen: T = 200 °C, p(H₂) = 80 bar, m(M) = 5 mg, m(Xylitol) = 1.00 g, m(Ca(OH)₂) = 0.150 g, 10 mL H₂O);
Figur 5 die Temperatur und Druckvariation für Ru/N-900-C (Bedingungen: m(Ru) = 5 mg, m(Xylitol) = 1 g, m(Ca(OH)₂) = 0.150 g, 10 mL H₂O);
Figur 6 die Hydrogenolyse von Sorbitol über N-900,5-Ru (Bedingungen: T = 200 °C, p(H₂) = 80 bar, m(Ru) = 5 mg, m(Sorbitol) = 1.20 g, m(Ca(OH)₂) = 0.150 g, 10 mL H₂O).

### Beispiel 1: Herstellung stickstoffhaltiger Kohlenstoffträger(N-C)

Das Beispiel illustriert die Herstellung stickstoffdotierter Kohlenstoffträger. 5 g Aktivkohle werden mit 35 mL HNO₃ (30%) versetzt und 8 h refluxiert. Die Kohle wird anschließend mit Wasser neutral gewaschen und getrocknet. 1 g der oxidierten Kohle wird in einen 50 mL Autoklaven gegeben, der mit 8 bar NH₃ und 52 bar N₂ beschickt wird. Der Autoklav wird unter Rühren auf 200 °C erhitzt. Die Reduktion der Kohle erfolgt über 4 h. Für den beschriebenen Kohleträger (N-HNO₃) ergibt sich ein Stickstoffgehalt von 5.54 % laut CHN Analyse. Wird der Träger anschließend noch mit Wasserstoff für 7 h bei 350 °C reduziert (N-HNO₃, H₂), ergibt sich ein Stickstoffgehalt von 4.65 %. Auf diesem Weg wird allerdings auch der Anteil an Sauerstoff auf der Kohle reduziert. Die Summe von C, H und N ergibt nun 91.50 %, während sie im Fall von N-HNO₃ bei 84.62 % liegt.

Einen anderen Weg, Kohlenstoffträger mit Stickstoff zu dotieren, stellt die Reduktion mit gasförmigem Ammoniak dar. Es werden Temperaturen zwischen 600 und 900 °C und Zeiten von 1 bis 5 h für die Reduktion gewählt und verschiedene Kohlen erhalten. Auch die Verwendung kommerzieller stickstoffdotierter Carbon Nanotubes (N-CNT) ist möglich. Vor Verwendung werden die NCNTs mit 10 Gew.-% HCl 2 h unter Rückfluss erhitzt. Anschließend werden sie mit Wasser neutral gewaschen und getrocknet. Eine Übersicht über die erhaltenen Stickstoffgehalte für die verschiedenen Kohleträger ist in Tabelle 1 gegeben.

### Beispiel 2: Imprägnierung des Trägers

Das Beispiel illustriert die Beladung eines Stickstoffhaltigen Kohleträgers mit einem Edelmetall. Beispielhaft wird Ruthenium verwendet. Je 500 mg der hergestellten Kohleträger werden zusammen mit 75.72 mg Dichloro(*p-*cymene)ruthenium(II) dimer in 145 mL Ethanol gegeben und unter Schutzgas bei 60 °C im Ölbad koordiniert. Die Koordinierung wird nach 71 Stunden abgebrochen und der Katalysator abfiltriert. Die maximal mögliche Beladung mit Ruthenium beträgt nach diesem Verfahren 5 Gew.-%. Das nicht koordinierte Ruthenium im Lösungsmittel wird mittels ICP MS untersucht und daraus rechnerisch die Beladung des Katalysators ermittelt. Die Beladung korreliert nicht mit dem Stickstoffgehalt und kann in Tabelle 1 eingesehen werden.

### Beispiel 3: Hydrogenolyse von Xylitol

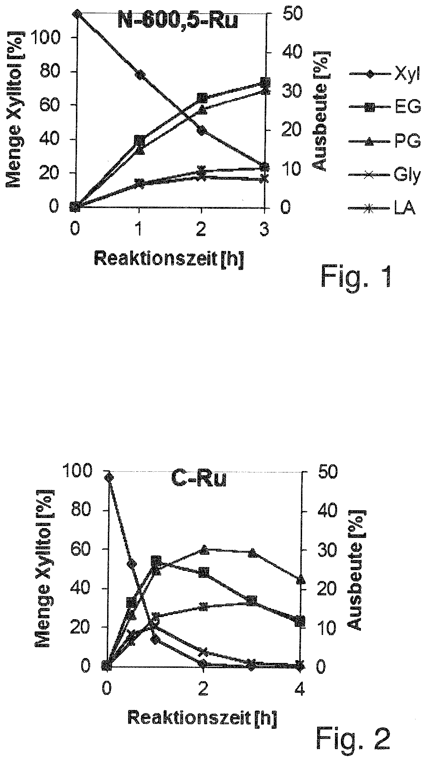

Das Beispiel illustriert beispielhaft die Hydrogenolyse von Zuckern und Zuckeralkoholen anhand der Verwendung von Xylitol (Xyl). Die Hydrogenolyse findet bei 200 °C und 80 bar Wasserstoffdruck in einem 50 mL Autoklaven statt. Es werden 1.50 g Xylitol, 0.225 g Ca(OH)₂ und 15 mL Wasser in den Autoklaven gegeben. Außerdem wird so viel Katalysator hinzugegeben, dass sich 7.5 mg Ru in der Reaktionslösung befinden. Für den Katalysator N-800,1-Ru ergibt sich somit eine Menge von 0.1563 g, für Ru/C (C-Ru) 0.1500 g. Die Reaktion erfolgte über 3 bis 4 h. In regelmäßigen Abständen wurden Proben genommen, um eine Kinetik zu erhalten. Die Produktentwicklung über die Zeit für N-600,5-Ru und C-Ru ist in den Fig. 1 und 2 gezeigt. Die gewünschten Produkte Ethylenglykol (EG) und Propylenglykol (PG) werden als Hauptprodukte gebildet. Glycerin (Gly) und Milchsäure (LA) fallen als Nebenprodukte nur in geringen Mengen an.

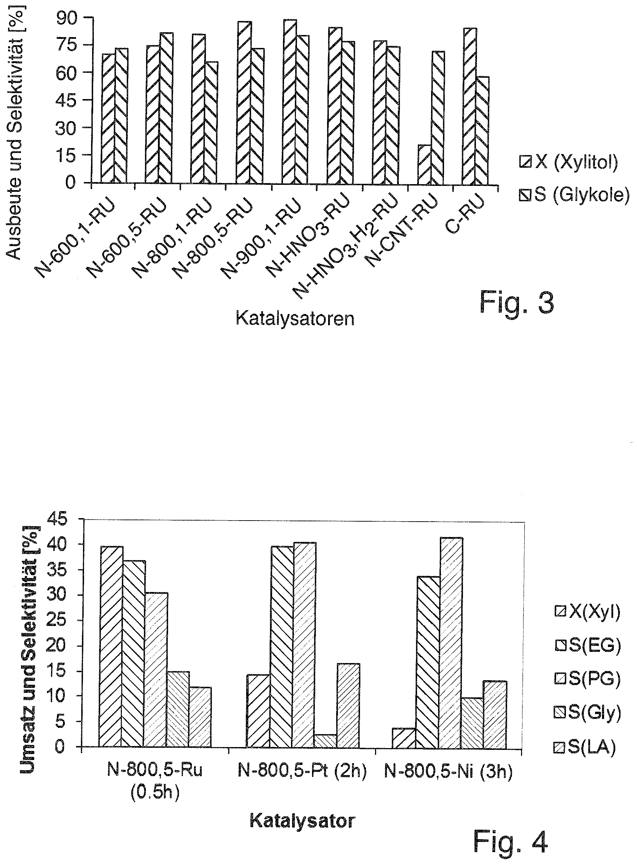

Für alle stickstoffhaltigen Katalysatoren werden unter diesen Bedingungen EG (Ethylenglykol) und PG (Propylenglykol) als Hauptprodukte gebildet. Die Summe der beiden Selektivitäten (S(Glykole)) erreicht in jedem Fall über 67 % für stickstoffdotierte Träger. Für C-Ru ist allerdings ein Abbau der Produkte bei längerer Reaktionszeit durch unerwünschte Nebenreaktionen erkennbar (Figur 2). Ein Vergleich der Katalysatoren ist in Figur 3 gezeigt. Die maximal erhaltene Glykol Selektivität beträgt 83 % (N-600,5-Ru). Zahlenwerte können Tabelle **1** entnommen werden.

**Tabelle 1. Erstellte Kohleträger, Stickstoffgehalt, Metallbeladung und Hydrogenolyseergebnisse.**

| **Kobleträger** | **Stickstoffgebalt/%** | **Ru-Beladung/Gew.-%** | **Reaktlenszelt/h** | **S(EG)/%** | **S(PG)/%** | **X(Xyl)/%** |
|---|---|---|---|---|---|---|
| N-600,1 | 0.49 | 4.43 | 3 | 38 | 36 | 70 |
| N-600,5 | 0.56 | 4.80 | 3 | 43 | 40 | 76 |
| N-800,1 | 1.64 | 3.36 | 1 | 37 | 30 | 52 |
| N-800,5 | 1.91 | 3.35 | 2 | 38 | 36 | 89 |
| N-900,1 | 1.41 | 1.36 | 3 | 41 | 41 | 94 |
| N-900,5 | 1.11 | 5.00 | - | - | | - |
| N-HNO₃ | 5.54 | 4.80 | 2 | 38 | 40 | 86 |
| N-HNO₃,H₂ | 4.65 | 3.85 | 3 | 36 | 38 | 91 |
| N-CNT | 4.20 | 4.99 | 1 | 32 | 42 | 21 |
| C | 0.00 | 5.00 | 1 | 31 | 29 | 86 |

### Beispiel 4: Metallscreening auf stickstoffhaltigen Kohlen

Es werden die Metalle Ni, Pt und Ru verglichen. Sie wurden auf den Träger N-800,5 geladen. Die Imprägnierung erfolgte äquivalent zu Beispiel 2. Nach Beladung wurden N-800,5-Pt und N-800,5-Ni im Wasserstoffstrom reduziert. Die Reduktion erfolgte bei 350 °C für 7 h. Die Hydrogenolyse erfolgte äquivalent zu Beispiel 3. Die Ergebnisse sind in Figur 4 und Tabelle **2** dargestellt. Es ist deutlich erkennbar, dass die katalytische Aktivität für N 800,5-Pt und N-800,5-Ni abnimmt, dennoch sind die erhaltenen Selektivitäten zu Glykolen unverändert hoch.

**Tabelle 2. Metallscreening auf stickstoffhaltigen Kohlen - Metallbeladung, Hydrogenolyseergebnisse.**

| **Katalysator** | **Metallbeladung/ %** | **Reaktionszeit/h** | **S(EG)/%** | **S(PG)/%** | **X(Xyl*)*/%** |
|---|---|---|---|---|---|
| N-800,5-Ru | 3.35 | 0.5 | 37 | 31 | 39 |
| N-800,5-Pt | 4.99 | 2 | 40 | 41 | 14 |
| N-800,5-Ni | 2.41 | 3 | 34 | 42 | 4 |

### Beispiel 5: Literaturvergleich

Die Ergebnisse der Hydrogenolyse von Zuckern und Zuckeralkoholen aus der vorliegenden Erfindung werden im Folgenden mit anderen Katalysatoren und Prozessen aus dem einschlägigen Stand der Technik in Bezug auf die Produktselektivität und Katalysatoraktivität verglichen. Dabei erfolgt der Vergleich bei ähnlichen Bedingungen und mit ähnlichen Substraten (siehe Tabelle 3).

**Tabelle 3. Vergleich vorliegende Erfindung mit dem Stand derTechnik.**

| **Ref** | **Substrat** | **Katalysator** | **Base** | **Kat:Sub** | **T/°C** | **p(H₂)/bar,RT** | **t/h** | **X/%** | **S(Glykole)/%** |
|---|---|---|---|---|---|---|---|---|---|
| [1] | Sorbitol | Ni-Re/C | KOH | 1:10 | 220 | 83 | 4 | 56 | 46 |
| [2] | Xylitol | Ru/C | KOH | 1:0.8 | 230 | 12 | - | 45 | 73 |
| [3] | Xylitol | Ni-Re/C | KOH | 1:10 | 200 | 83 | - | 50 | 65 |
| * | Xylitol | Ru/N-C | Ca(OH)2 | 1:10 | 200 | 80 | 3 | 76 | 83 |

| | | | | | | | | | |
|---|---|---|---|---|---|---|---|---|---|
| *Vorliegende Erfindung | | | | | | | | | |

[1] T. Werpy, J. Frye, A. Zacher, D. Miller, US20030119952 A1, 2003. [2] S. P. Chopade, D.J. Miller, J. E. Jackson, T. A. Werpy, J. G. Frye, Jr., A. H. Zacher, WO2001066499, 2001. [3] J. G. Frye, D. J. Miller, T. A. Werpy, A. H. Zacher, WO2003035593 B1, 2003.

### Beispiel 6: Temperatur und Druckvarlation Für Ru/N-C

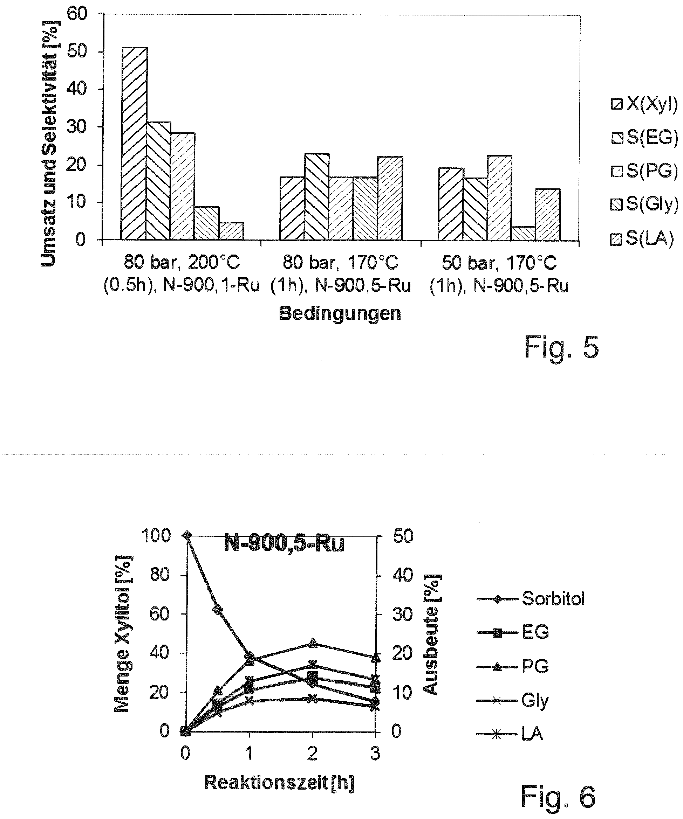

Für den Katalysator N-900-Ru wurde eine Variation der Temperatur und des Drucks durchgeführt. Neben 200 °C wurden auch 170 °C verwendet, neben 80 bar H₂ wurden auch 50 bar H₂ verwendet. Die Hydrogenolyse wurde äquivalent zu Beispiel 3 durchgeführt und die Ergebnisse sind vergleichend in Figur 5 und Tabelle 4 dargestellt. Für geringere Temperaturen verläuft die Reaktion langsamer. Ein Vergleich der Selektivitäten fällt schwer, da nicht bei gleichem Umsatz verglichen werden kann. Dennoch bleiben Glykole die Hauptprodukte der Reaktion.

**Tabelle4. Erstellte Kohleträger, Stickstoffgehalt, Metallbeladung und Hydrogenoylseergebnisse.**

| **Katalysator** | **Reaktionszeit/h** | **p/h** | **T/°C** | **S(EG)/%** | **S(PG)/%** | **X(Xyl)/%** |
|---|---|---|---|---|---|---|
| N-900,1-Ru | 0.5 | 80 | 200 | 31 | 28 | 51 |
| N-900,5-Ru | 1 | 80 | 170 | 23 | 17 | 17 |
| N-900,5-Ru | 1 | 50 | 200 | 17 | 23 | 20 |

### Beispiel 7: Hydrogenolyse von Sorbitol

Das Beispiel illustriert die Hydrogenolyse von Zuckern und Zuckeralkoholen anhand der Verwendung von Sorbitol (Sor). Die Hydrogenolyse findet bei 200 °C und 80 bar Wasserstoffdruck in einem 50 mL Autoklaven statt. Es werden 1.197 g Sorbitol, 0.150 g Ca(OH)₂ und 10 mL Wasser in den Autoklaven gegeben. Außerdem wird so viel Katalysator hinzugegeben, dass sich 5 mg Ru in der Reaktionslösung befinden. Für den Katalysator N-900,5-Ru ergibt sich somit eine Menge von 0.100 g. Die Reaktion erfolgte über 3 h. In regelmäßigen Abständen wurden Proben genommen, um eine Kinetik zu erhalten. Die Produktentwicklung über Zeit für N-900,5-Ru ist in Figur 6 gezeigt. Die Selektivität zu EG nach 2 h Reaktion beträgt 18 %, die Selektivität zu PG 30 %.

Es wurden im Rahmen der vorliegenden Erfindung Vergleichsversuche durchgeführt, bei denen Carbon Nanotubes als Katalysatorträger eingesetzt wurden, die nicht mit Stickstoff dotiert waren. Dabei wurde unter vergleichbaren Bedingungen gearbeitet wie in den oben genannten erfindungsgemäßen Beispielen. Es wurde festgestellt, dass dabei die erfindungsgemäßen Katalysatorträger überlegen sind, da sie einen Einfluss auf die Selektivitäten der Zielprodukte aufweisen.

Es wurden im Rahmen der vorliegenden Erfindung Vergleichsversuche durchgeführt, bei denen Aktivkohle als Katalysatorträger eingesetzt wurde, die nicht mit Stickstoff dotiert war. Dabei wurde unter vergleichbaren Bedingungen gearbeitet wie in den oben genannten erfindungsgemäßen Beispielen. Es wurde festgestellt, dass dabei die erfindungsgemäßen Katalysatorträger überlegen sind, da die Stickstoffdotierung einen drastischen, positiven Einfluss auf die Selektivitäten der Zielprodukte aufweist. Des Weiteren wird ein Abbau der Produkte (z.B. im Falle von Ru/C vs. Ru/N-C) durch die Stickstoffdotierung des Kohlenstoffträgers deutlich verlangsamt/verringert.

## Patentansprüche

1. Verfahren zur chemischen Umsetzung von Zuckern oderZuckeralkoholen zu Polyolen/Glykolen wobei die Umsetzung der Zucker oder Zuckeralkohole durch Hydrogenolyse in Gegenwart eines mindestens ein Metall enthaltenden Katalysators auf einem Kohlenstoff-Träger erfolgt, **dadurch gekennzeichnet, dass** als Katalysator-Träger ein mit Stickstoff dotierter Kohlenstoff-Träger verwendet wird.

2. Verfahren nach Anspruch 1, **dadurch gekennzeichnet, dass** man in einem zweistufigen Prozess zunächst einen Zucker zu einem Zuckeralkohol hydriert und danach in einem zweiten Schritt den Zuckeralkohol durch Hydrogenolyse zu Polyolen umsetzt.

3. Verfahren nach Anspruch 1 oder 2, **dadurch gekennzeichnet, dass** man einen C6-Zucker oder einen C6-Zuckeralkohol oder einen C5-Zucker oder einen C5-Zuckeralkohol durch Hydrierung/Hydrogenolyse zu Polyolen/Glykolen umsetzt.

4. Verfahren nach einem der Ansprüche 1 bis 3, **dadurch gekennzeichnet, dass** als Katalysator-Träger mit Stickstoff dotierte Aktivkohle oder mit Stickstoff dotierter Ruß verwendet wird.

5. Verfahren nach Anspruch 4, **dadurch gekennzeichnet, dass** als Katalysator ein Kohlenstoffträger verwendet wird, dessen Oberfläche mittels reduktiver Methoden, insbesondere mit Hilfe von Ammoniak und/oder Stickstoff und/oder Wasserstoff mit Stickstoffatomen dotiert wurde.

6. Verfahren nach einem der Ansprüche 1 bis 3, **dadurch gekennzeichnet, dass** als Katalysator-Träger mit Stickstoff dotierte Carbon Nanotubes verwendet werden.

7. Verfahren nach Anspruch 6, **dadurch gekennzeichnet, dass** als Cabon Nanotubes zylinderförmige Kohlenstoffhohlkörper mit einem Durchmesservon 0,4 bis 100 nm verwendet werden,, die während ihrer Herstellung zusätzlich durch Stickstoff dotiert wurden.

8. Verfahren nach einem der Ansprüche 1 bis 7, **dadurch gekennzeichnet, dass** der Katalysator eines oder mehrere Metalle enthält, ausgewählt aus der Gruppe umfassend:
Ru, Pt, Ni, Os, Rh, Ir, Pd, sowie Au, Ni, Cu, Fe und Co.

9. Verfahren nach einem der Ansprüche 1 bis 8, **dadurch gekennzeichnet, dass** als Co-Katalysator eine Base verwendet wird.

10. Verfahren nach Anspruch 9, **dadurch gekennzeichnet, dass** als Base ein Alkalihydroxid oder ein Erdalkalihydroxid verwendet wird, insbesondere ausgewählt aus der Gruppe umfassend: (NaOH), KOH, LiOH, Mg(OH)₂, Ca(OH)₂, Sr(OH)₂ und Ba(OH)₂.

11. Verfahren nach einem der Ansprüche 1 bis 10, **dadurch gekennzeichnet, dass** die Umsetzung bei einer Reaktionstemperatur im Bereich von 20 °C bis 400 °C, insbesondere im Bereich zwischen 170 °C bis 200 °C erfolgt.

12. Verfahren nach einem der Ansprüche 1 bis 11, **dadurch gekennzeichnet, dass** die Hydrogenolyse bei einem Wasserstoffdruck im Bereich von etwa 1 bar bis 300 bar, insbesondere im Bereich von 50 bar bis 80 bar, erfolgt.

## Claims

1. A method for chemically converting sugars or sugar alcohols into polyols/glycols, wherein the sugars or sugar alcohols are converted by means of hydrogenolysis in the presence of a catalyst comprising at least one metal and on a carbon support, **characterized in that** a nitrogen-doped carbon support is used as catalyst support.

2. The method as claimed in claim 1, **characterized in that,** in a two-stage process, firstly a sugar is hydrogenated to give a sugar alcohol and thereafter the sugar alcohol is converted into polyols in a second step by means of hydrogenolysis.

3. The method as claimed in claim 1 or 2, **characterized in that** a C6 sugar or a C6 sugar alcohol or a C5 sugar or a C5 sugar alcohol is converted by means of hydrogenation/hydrogenolysis into polyols/glycols.

4. The method as claimed in any of claims 1 to 3, **characterized in that** nitrogen-doped activated carbon or nitrogen-doped carbon black is used as catalyst support.

5. The method as claimed in claim 4, **characterized in that** a carbon support is used as catalyst, the surface of which was doped with nitrogen atoms by means of reductive methods, especially using ammonia and/or nitrogen and/or hydrogen.

6. The method as claimed in any of claims 1 to 3, **characterized in that** nitrogen-doped carbon nanotubes are used as catalyst support.

7. The method as claimed in claim 6, **characterized in that** as carbon nanotubes use is made of cylindrical carbon hollow bodies having a diameter of 0.4 to 100 nm which were additionally doped with nitrogen during the production thereof.

8. The method as claimed in any of claims 1 to 7, **characterized in that** the catalyst comprises one or more metals selected from the group comprising:
Ru, Pt, Ni, Os, Rh, Ir, Pd, and also Au, Ni, Cu, Fe and Co.

9. The method as claimed in any of claims 1 to 8, **characterized in that** a base is used as cocatalyst.

10. The method as claimed in claim 9, **characterized in that** an alkali metal hydroxide or an alkaline earth metal hydroxide is used as base, especially selected from the group comprising: (NaOH), KOH, LiOH, Mg(OH)₂, Ca(OH)₂, Sr(OH)₂ and Ba(OH)₂.

11. The method as claimed in any of claims 1 to 10, **characterized in that** the conversion is effected at a reaction temperature in the range from 20°C to 400°C, especially in the range between 170°C to 200°C.

12. The method as claimed in any of claims 1 to 11, **characterized in that** the hydrogenolysis is effected at a hydrogen pressure in the range from approximately 1 bar to 300 bar, especially in the range from 50 bar to 80 bar.

## Revendications

1. Procédé pour la transformation chimique de sucres ou d'alcools de sucres en polyols/glycols, la transformation des sucres ou alcools de sucres ayant lieu par hydrogénolyse en présence d'un catalyseur contenant au moins un métal sur un support carboné, **caractérisé en ce qu'**un support carboné dopé avec de l'azote est utilisé en tant que support de catalyseur.

2. Procédé selon la revendication 1, **caractérisé en ce que,** dans un processus à deux étapes, un sucre est tout d'abord hydrogéné en un alcool de sucre et ensuite, dans une deuxième étape, l'alcool de sucre est transformé en polyols par hydrogénolyse.

3. Procédé selon la revendication 1 ou 2, **caractérisé en ce qu'**un sucre en C6 ou un alcool de sucre en C6 ou un sucre en C5 ou un alcool de sucre en C5 est transformé en polyols/glycols par hydrogénation/hydrogénolyse.

4. Procédé selon l'une quelconque des revendications 1 à 3, **caractérisé en ce que** du charbon actif dopé avec de l'azote ou du noir de carbone dopé avec de l'azote est utilisé en tant que support de catalyseur.

5. Procédé selon la revendication 4, **caractérisé en ce qu'**un support carboné est utilisé en tant que catalyseur, dont la surface a été dopée avec des atomes d'azote au moyen de méthodes de réduction, notamment à l'aide d'ammoniac et/ou d'azote et/ou d'hydrogène.

6. Procédé selon l'une quelconque des revendications 1 à 3, **caractérisé en ce que** des nanotubes de carbone dopés avec de l'azote sont utilisés en tant que support de catalyseur.

7. Procédé selon la revendication 6, **caractérisé en ce que** des corps creux carbonés cylindriques ayant un diamètre de 0,4 à 100 nm sont utilisés en tant que nanotubes de carbone, qui ont en outre été dopés par de l'azote pendant leur fabrication.

8. Procédé selon l'une quelconque des revendications 1 à 7, **caractérisé en ce que** le catalyseur contient un ou plusieurs métaux, choisis dans le groupe comprenant :
Ru, Pt, Ni, Os, Rh, Ir, Pd, ainsi qu'Au, Ni, Cu, Fe et Co.

9. Procédé selon l'une quelconque des revendications 1 à 8, **caractérisé en ce qu'**une base est utilisée en tant que co-catalyseur.

10. Procédé selon la revendication 9, **caractérisé en ce qu'**un hydroxyde alcalin ou un hydroxyde alcalino-terreux est utilisé en tant que base, notamment choisi dans le groupe comprenant : (NaOH), KOH, LiOH, Mg(OH)₂, Ca(OH)₂, Sr(OH)₂ et Ba(OH)₂.

11. Procédé selon l'une quelconque des revendications 1 à 10, **caractérisé en ce que** la transformation a lieu à une température de réaction dans la plage allant de 20 °C à 400 °C, notamment dans la plage allant de 170 °C à 200 °C.

12. Procédé selon l'une quelconque des revendications 1 à 11, **caractérisé en ce que** l'hydrogénolyse a lieu à une pression d'hydrogène dans la plage allant d'environ 1 bar à 300 bar, notamment dans la plage allant de 50 bar à 80 bar.
